# EUROPEAN PATENT APPLICATION

(11) **EP 4 306 641 A1**
(43) Date of publication of application: **17.01.2024**
(21) Application number: 22750088.1
(22) Date of filing: 07.02.2022
(51) Int. Cl.: C12N 15/62, C07K 14/005, C07K 16/00, C07K 14/33, A61K 39/215, A61P 31/14, A61P 35/00, A61K 47/50

(54) **NOVEL NUCLEIC ACID MOLECULE**

(30) Priority: 05.02.2021 KR 20210017115
(71) Applicant: ST Pharm Co., Ltd., Siheung-si, Gyeonggi-do 15086 (KR)
(72) Inventor: KIM, Kyungjin, Seoul 06194 (KR); YANG, Joo-Sung, Seoul 06517 (KR); CHOI, Kang Hyun, Suwon-si Gyeonggi-do 16387 (KR); LEE, Yun Chae, Siheung-si Gyeonggi-do 15011 (KR)
(74) Representative: Rückerl, Florian
(86) International application number: PCT/KR2022/001874
(87) International publication number: WO 2022/169339

(57) **Abstract**

The present invention relates to a nucleic acid molecule for preventing or treating viral infection or cancer, comprising a nucleic acid encoding a signal peptide and a nucleic acid encoding an antigen. Furthermore, it relates to a vaccine composition for preventing or treating viral infection or cancer, comprising the nucleic acid molecule.

The nucleic acid molecule according to the present invention has excellent intracellular protein expression rate and protein secretion ability to the outside of the cell. In addition, when administered in vivo, it allows the subject to acquire humoral immunity, such as inducing antigen-specific neutralizing antibodies, and cellular immunity, such as increasing the amount of immune cells directly involved in killing the virus. Thus, it can be useful as a vaccine for the prevention and treatment of viral infection or cancer.

## Description

### [Technical Field]

The present invention relates to a nucleic acid molecule for preventing or treating viral infection or cancer, comprising a nucleic acid encoding a signal peptide and a nucleic acid encoding an antigen. Furthermore, it relates to a vaccine composition for preventing or treating viral infection or cancer, comprising the nucleic acid molecule.

### [Background Art]

Vaccines are currently developed and manufactured in a variety of ways, and specific types include attenuated vaccines which inject pathogens in an attenuated state; toxoid vaccines which are made by inactivating the causative agent (toxin) that causes the disease, not the pathogen itself; subunit vaccines which are made by extracting only the portions corresponding to antigenic determinants recognized as antigens; and recombinant vaccines which are made by separately producing only the epitope using genetic information.

Among the above-mentioned vaccines, the recombinant vaccines have the advantage of being highly safe since they do not contain components other than epitopes. In particular, mRNA-based nucleic acid vaccines offer many advantages of rapid production, cost-effectiveness, and the ability to induce immune responses for both cell-mediated immunity and humoral immunity. Additionally, it can be designed in a variety of ways, such as by selecting and using only specific epitopes that induce an immune response among the various components of the antigen, and accordingly it is useful in the prevention or treatment of various diseases such as viral infections and cancer.

For example, viral infections range from relatively mild symptoms such as cold, influenza, chickenpox and herpes labialis to severe diseases causing serious side effects and sequelae such as rabies, Ebola, Marburg, Crimean-Congo hemorrhagic fever, AIDS, avian influenza, SARS, COVID-19, cancer, etc. Accordingly, preventing the onset of symptoms rather than alleviating and treating symptoms after infection is a major issue, and thus vaccines for the prevention of viral infections are actively developed.

In addition, cancer vaccines are being researched and developed for the prevention and treatment of not only cancer caused by viral infection, but also cancer caused by cell mutation. Cancer cells contain tumor-specific antigens that do not exist in normal cells. Cancer vaccines kill cancer cells before they proliferate excessively by inducing a cancer cell-specific immune response through the tumor-specific antigens. Some cancer vaccines are tailored to individual patients. By analyzing the patient's cancer cell genes, the mutant genes that cause cancer are identified, and vaccines are manufactured using mRNA translated into neoantigen epitopes encoded by these genes. This approach allows for the development of personalized precision medicine, as they can induce potent immune responses specific to the patient.

However, due to the characteristics of the mechanism of action of nucleic acid vaccines such as mRNA vaccines, the efficiency of protein expression is very important, and the efficacy of the vaccine is determined by the level of protein expression. Therefore, there is a need for the development of substances that not only target various diseases but also have improved protein expression capabilities.

### [Technical Problem]

The purpose of the present invention is to provide a nucleic acid molecule for preventing or treating viral infections or cancer, comprising a nucleic acid encoding a signal peptide and a nucleic acid encoding an antigen, and a vaccine composition comprising the nucleic acid molecule.

### [Technical Solution]

Each description and embodiment disclosed in the present invention may be also applied to other descriptions and embodiments. That is, all combinations of the various elements disclosed in the present invention fall within the scope of the present invention. Additionally, the scope of the present invention cannot be considered limited by the specific description described below.

Additionally, terms that are not specifically defined in this specification should be understood to have meanings commonly used in the technical field to which the present invention pertains. Additionally, unless specifically defined by context, the singular includes the plural, and the plural includes the singular.

One aspect of the present invention provides a nucleic acid molecule for preventing or treating viral infection or cancer, comprising a nucleic acid encoding a signal peptide and a nucleic acid encoding an antigen.

The term "signal peptide" refers to a peptide present at the N-terminus of a protein at the beginning of protein synthesis, which is also called signal sequence, targeting signal, localization signal, localization sequence, leader sequence, or leader peptide. The signal peptide according to the present invention can play a role in increasing protein expression and secretion of nucleic acid molecules. In this specification, the term 'protein expression' or 'protein expression rate' may be used interchangeably with the same meaning as 'protein translation', 'protein translation rate', 'antigen expression', and 'antigen expression rate'.

Specifically, the signal peptide may be derived from one or more selected from the group consisting of Immunoglobulin E (IgE), albumin, interferon gamma (IFN-γ), factor IX, and mucin-like protein 1 (MLP1), but is not limited thereto.

In addition, the signal peptide may be one or more selected from the group consisting of the peptides represented by SEQ ID NO: 1 derived from IgE, SEQ ID NO: 5 derived from albumin, SEQ ID NO: 9 derived from IFN-γ, SEQ ID NO: 13 derived from factor IX, and SEQ ID NO: 17 derived from MLP1, but is not limited thereto.

Additionally, the nucleic acid encoding the signal peptide may be one or more selected from the group consisting of SEQ ID NOs: 2 to 4, 6 to 8, 10 to 12, 14 to 16, and 18 to 20, but is not limited thereto.

The term "antigen" refers to a protein that triggers an immune response such as specific antibody production and cytokine secretion, and may be derived from one or more selected from the group consisting of coronavirus, oncovirus, tumor-specific antigen, tumor-associated antigen and neoantigen epitope.

Specific examples of the coronavirus may include alphacoronavirus (α-CoV, alphaCoV), betacoronavirus (β-CoV, betaCoV), gammacoronavirus (γ-CoV, gammaCoV), or deltacoronavirus (δ-CoV, deltaCoV), and more specifically, it may be, but not limited to, HCoV-229E, HCoV-NL63, Bat-SARS-like(SL)-ZC45, Bat-SL ZXC21, SARS-CoV, MERS-CoV, HCoV-OC43, HKU-1, MHV-A59, or SARS-CoV-2.

More specifically, the antigen may be one or more selected from the group consisting of spike protein and membrane protein derived from coronavirus.

The spike protein of the coronavirus according to the present invention can play a role in inducing an immune response and increasing the ability to produce neutralizing antibodies, and the membrane protein can induce an immune response, such as improving the function of CD4+ Th cells, to kill virus-infected cells.

The oncovirus refers to a virus that causes tumors or cancer, and specific examples include HBV (Hepatitis B virus), HCV (Hepatitis C virus), HTLV (Human T-lymphotropic virus), HPV (Human papillomaviruses), HHV-8 (Kaposi's sarcoma-associated herpesvirus), MCV (Merkel cell polyomavirus), or EBV (Epstein-Barr virus), but is not limited thereto.

In addition, the terms 'tumor-specific antigen', tumor-associated antigen', and 'neoantigen epitope' refer to antigens or epitopes that do not exist in normal cells but exist only in cancer cells. Specific examples may include mutant genes caused by cancer cell-specific mutations such as mutational frameshift, splice variant, gene fusion, and endogenous retroelement, or mRNAs or proteins expressed from the mutant genes.

Additionally, the antigen may have one or more amino acid sequences selected from the group consisting of SEQ ID NOs: 21 and 26; and the nucleic acid encoding the antigen may have one or more nucleic acid sequences selected from the group consisting of SEQ ID NOs: 22 to 25, and 27 to 30, but is not limited thereto.

The nucleic acid molecules according to the invention may comprise nucleic acids encoding Th cell epitopes.

The term `Th cell epitope' refers to a peptide that is recognized by Th cells and induces an immune response by Th cells, and the `T helper cell (Th cell)' refers to immune cells also called CD4+ cells, CD4+ T cells, T helper lymphocytes, or helper T cells. The Th cell epitope according to the present invention can play a role in increasing the ability to produce neutralizing antibodies by inducing an immune response by Th cells, specifically, secreting various cytokines such as IFN-γ, TNF-α, IL-2, IL-10, IL-4, IL-5, IL-6, IL-9, IL-10, IL-13 to carry out immune responses such as antibody production of B cells, activation of cytotoxic T cells, activation of memory T cells, and promotion of antibacterial activity of phagocytes including macrophages.

Specifically, the Th cell epitope may be a peptide derived from one or more selected from the group consisting of Tetanus toxoid, diphtheria toxoid (DTH toxoid), and Purtussis toxoid. The toxoid refers to a substance in which toxicity has been removed but immunogenicity, which induces an immune response, remains. `Tetanus toxoid' may be derived from tetanospasmin, a neurotoxin produced by the tetanus bacterium *Clostridium tetani.* `Diphtheria toxoid (DTH Toxoid)' may be derived from an exotoxin produced by *Corynebacterium diphtheria.* 'Pertussis toxoid' may be derived from peptidoglycan fragments produced by the pertussis bacterium *Bordetella pertussis.* In addition, the Th cell epitope may include epitopes composed of peptide sequences that are presented by the MHC Class II of antigen-presenting cells such as dendritic cells or macrophages and are detected by the T-cell receptor of Th cells to transmit a stimulatory signal.

In addition, the Th cell epitope may further comprise a nucleic acid encoding a cleavage site, and may further comprise a nucleic acid sequence encoding a cleavage site between the antigen and the Th cell epitope. The term 'cleavage site' refers to a peptide that is cleaved by a proteolytic enzyme. In the present invention, it serves to ensure that the protein expressed from the nucleic acid molecule is cleaved at the site recognized by the enzyme, and accordingly it allows each signal peptide, antigen and/or Th cell epitope linked to the nucleic acid molecule to play the role according to the present invention. The cleavage site may be cleaved by an endogenous enzyme present in the cell, and specifically may be cleaved by a furine protease or a 2A self-cleaving peptide, but is not limited thereto.

Additionally, the Th cell epitope may be the amino acid sequence of SEQ ID NO: 44; the nucleic acid encoding the Th cell epitope may be one or more nucleic acid sequences selected from the group consisting of SEQ ID NOs: 45 and 46, but is not limited thereto.

The term "viral infection" refers to a disease that occurs as a result of the proliferation of viruses in the organs or tissues of the human body.

Specifically, the viral infection may be a coronavirus infection. Specific examples of the coronavirus infection may be one or more selected from the group consisting of Severe Acute Respiratory Syndrome (SARS), Middle East Respiratory Syndrome (MERS), and Coronavirus disease-2019 (COVID-19), but is not limited thereto.

Additionally, the viral infection may be cancer. Specific examples of the cancer may be one or more selected from the group consisting of solid tumor and blood cancer. The solid tumor may include hepatocarcinoma, cervical cancer, anal cancer, penis cancer, vulva cancer, vaginal cancer, oropharyngeal cancer, Kaposi's sarcoma, nasopharyngeal carcinoma, stomach cancer, multicentric Castleman's disease, Merkel cell carcinoma, etc., and the blood cancer may include T-cell leukemia, primary effusion lymphoma, Burkitt's lymphoma, Hodgkin's lymphoma, post-transplant lymphoproliferative disease, etc. However, it is not limited thereto as long as it is caused by oncovirus infection.

In addition, specific examples of cancer that can be prevented by the nucleic acid molecule according to the present invention may be one or more selected from the group consisting of solid tumor and blood cancer. The solid tumor may be, but not limited to, stomach cancer, lung cancer, liver cancer, colorectal cancer, colon cancer, kidney cancer, small intestine cancer, pancreatic cancer, brain tumor, bone cancer, skin cancer, epidermoid carcinoma, squamous cell carcinoma, breast cancer, sclerosing gonadosis, head and neck cancer, esophagus cancer, pharynx cancer, thyroid cancer, parathyroid cancer, neuroblastoma, melanoma, sarcoma, prostate cancer, uterine cancer, cervical cancer, ovarian cancer, vaginal cancer, vulvar cancer, urethral cancer, bladder cancer, penile cancer, testicular cancer, fibroadenoma, or metastatic cancer thereof. The blood cancer may be, but not limited to, hematological cancer, angiosarcoma, leukemia, lymphoma, or metastatic cancer thereof.

Additionally, the nucleic acid molecule according to the present invention may be a nucleic acid molecule for preparing a vaccine, or a nucleic acid molecule for preparing a vaccine for preventing or treating viral infection or cancer.

The term 'prevention' refers to any action in which viral infection or cancer is inhibited or delayed by the nucleic acid molecule according to the present invention.

The term "treatment" refers to any action in which symptoms of a viral infection or cancer are improved or completely cured by the nucleic acid molecule according to the present invention.

The term "nucleic acid molecule" is meant to comprehensively include DNA and RNA molecules. A nucleotide, which is the basic structural units in the nucleic acid molecule, includes not only natural nucleotides but also analogues in which sugar or base sites are modified. The sequence of a nucleic acid molecule of the invention may be mutated, and the mutation includes the addition, deletion, non-conservative substitution, or modification of one or more nucleotides.

In addition, all sequences used in the present invention, including nucleic acid sequences and amino acid sequences, are interpreted to include sequences showing substantial identity with the sequences included in the sequence list, considering mutations with biologically equivalent activity. The term 'substantial identity' means that when the sequence of the present invention and any other sequence are aligned to the maximum extent possible and the aligned sequence is analyzed using an algorithm commonly used in the art, it refers to a sequence showing at least 60% homology, more specifically 70% homology, even more specifically 80% homology, most specifically 90%, 91%, 92%, 93%, 94%, 95%, 96%, 97%, 98% or 99% homology.

Therefore, sequences having high homology to the sequences represented by SEQ ID NOs. 1 to 59 of the present invention, for example, having a homology of 70% or more, specifically 80% or more, and more specifically 90% or more should also be construed as being included within the scope of the present invention.

The nucleic acid molecule may comprise a signal peptide, antigen, and/or Th cell epitope according to the present invention in the following structure: 5'-[signal peptide]-[antigen]-3' or 5'-[signal peptide]-[antigen]-[Th cell epitope]-3'.

Herein, the signal peptide, antigen and/or Th cell epitope are not limited to the above-described order, and there is no limitation to the order as long as they can perform the role according to the present invention.

Additionally, the signal peptide, antigen and/or Th cell epitope may be comprised in the nucleic acid molecule singly or plurally, and there is no limit to their number as long as the protein expression rate is not reduced.

Furthermore, the nucleic acid molecule may comprise, in addition to the signal peptide, antigen and/or Th cell epitope, a general nucleic acid sequence for expression into protein, for example, 5'-CAP containing 7-methylguanosine, Kozak sequence, initiation codon, termination codon, 3'-Poly A tail containing adenosine, etc.

Another aspect of the present invention provides a vaccine composition for preventing or treating viral infection or cancer, comprising the nucleic acid molecule.

The descriptions for `nucleic acid molecule', `viral infection', and 'cancer' are the same as described above.

The term 'prevention' refers to any action in which viral infection or cancer is inhibited or delayed by administration of the vaccine composition according to the present invention.

The term "treatment" refers to any action in which symptoms of a viral infection or cancer are improved or completely cured by administration of the vaccine composition according to the present invention.

The term 'vaccine' refers to a biological agent containing an antigen that provides immunity to a subject, and indicates an immunogen or antigenic substance that occurs immunity in the subject by injecting or orally administering it to the subject to prevent infection. The vaccine may be RNA vaccine, and specifically mRNA vaccine. The term `mRNA vaccine' refers to a vaccine that induces an immune response by artificially replicating some of the genes of an antigen and then administering it. These mRNA vaccines have various advantages over existing protein vaccines. First, because they can be synthesized using only the genetic information of the target antigen, there is no need to directly handle dangerous pathogens. They use only some of the genes required to induce toxicity, and thus there is no risk of significant toxicity even if administered. Because of their simplicity composed only of mRNA, it has various advantages such as the ability to quickly develop vaccines against infectious diseases or various mutations which are suddenly occurred.

Specifically, the vaccine composition for preventing or treating viral infection or cancer according to the present invention can be used for preventing or treating coronavirus infection and/or cancer, and can have immunity to coronavirus, oncovirus, tumor-specific antigen, tumor-associated antigen and/or neoantigen epitope. Specific examples of the coronavirus may include alphacoronavirus genus, betacoronavirus genus, gammacoronavirus genus or deltacoronavirus genus, more specifically HCoV-229E, HCoV-NL63, Bat-SARS-like(SL)-ZC45, Bat-SLZXC21, SARS-CoV, MERS-CoV, HCoV-OC43, HKU-1, MHV-A59, or SARS-CoV-2, and specific examples of the oncovirus may include HBV, HCV, HTLV, HPV, HHV-8, MCV or EBV, but is not limited thereto.

In addition, the nucleic acid molecule comprised in the vaccine composition may be encapsulated in or linked to a delivery vehicle, and the delivery vehicle may be, but not limited to, one or more selected from the group consisting of liposome-based delivery vehicle, lipid-based delivery vehicle, polymer-based delivery vehicle, and lipid-polymer hybrid nanoparticles. Specific examples of the carrier may include liposome-based or lipid-based delivery vehicles such as liposome, phytosome, ethosome, lipid nanoparticle, lipid-like nanoparticle, lipid emulsion, lipoplex and lipid micelle; polymer-based delivery vehicles such as polymersome, polymeric nanoparticle, dendrimer, nanosphere, polyplex, and polymeric micelle; or a lipid-polymer hybrid nanoparticle such as cationic nanoemulsion, anionic nanoemulsion, or lipopolyplex, but is not limited thereto.

The vaccine composition of the present invention may comprise a pharmaceutically acceptable carrier. The term 'pharmaceutically acceptable carrier' includes any and all solvents, dispersion media, coating agents, adjuvants, stabilizers, excipients, diluents, preservatives, antibacterial and antifungal agents, isotonic agents, adsorption delay agents, etc. Specific examples of the carrier may include, but are not limited to, lactose, dextrose, sucrose, sorbitol, mannitol, xylitol, maltitol, starch, glycerin, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil.

In addition, the vaccine composition of the present invention may be formulated and used in the oral dosage forms such as powders, granules, tablets, capsules, suspensions, emulsions, syrups, aerosols, etc., or sterile injectable solutions according to conventional methods. When formulating, it can be prepared using diluents or excipients such as fillers, extenders, binders, wetting agents, disintegrants and surfactants commonly used.

The vaccine composition may be administered to a subject in various forms. The term "administration" may be performed by subcutaneous administration, intramuscular administration, intradermal administration, intraperitoneal administration, intravenous administration, intranasal administration, transdermal administration, parenteral administration, and oral administration, but is not limited thereto.

The vaccine composition may comprise one or more adjuvants to improve or strengthen the immune response. Suitable adjuvants include synthetic analogs of doublestranded RNA (dsRNA), oligonucleotides of the unmethylated cytidine-guanidine type, peptides, aluminum hydroxide, aluminum phosphate, aluminum oxide and mineral or vegetable oils such as Marcol 52, and compositions composed of one or more emulsifiers or surface active substances such as lysolecithin, polyvalent cations, and polyanions.

Another aspect of the present invention provides a method for generating an immune response against viral infection or cancer in a subject, comprising administering the vaccine composition to the subject in need thereof.

The descriptions relating to `vaccine composition', 'viral infection', `cancer', and 'administration' are the same as described above.

The term 'subject' includes all individuals who are likely to be infected or are infected with a virus, or are likely to develop or have cancer, and may include, without limitation, humans, any non-human animal, fish, or plants. The non-human animal may be a vertebrate, such as a primate, dog, cow, horse, pig, rodent, such as mouse, rat, hamster, guinea pig, etc. In this specification, the 'subject' may be used interchangeably with 'individual' or 'patient'.

The term 'immune response' refers to the activation of a subject's immune system in response to the introduction of an antigen. The immune response may be in the form of cell-mediated immunity, humoral immunity, or both.

In the method for generating an immune response, the vaccine composition of the present invention may comprise an effective amount of the active ingredient, that is, the nucleic acid molecule according to the present invention, together with a pharmaceutically acceptable carrier and adjuvant. The term `effective amount' refers to an amount sufficient to induce a specific immune response against viral infection or cancer in the subject to which the vaccine composition is administered. The effective amount may be easily determined by a person skilled in the art, for example, through routine experiments in animals.

Another aspect of the present invention provides a method for preventing or treating a viral infection or cancer in a subject, comprising administering the vaccine composition to the subject in need thereof.

The descriptions relating to `vaccine composition', 'viral infection', `cancer', 'subject', 'administration', 'prevention' and 'treatment' are the same as described above.

The nucleic acid molecule according to the present invention has excellent intracellular protein expression rate and protein secretion ability to the outside of the cell. In addition, when administered in vivo, it allows the subject to acquire humoral immunity, such as inducing antigen-specific neutralizing antibodies, and cellular immunity, such as increasing the amount of immune cells directly involved in killing the virus. Thus, it can be useful as a vaccine for the prevention and treatment of viral infection or cancer.

### [Brief Description of Figures]

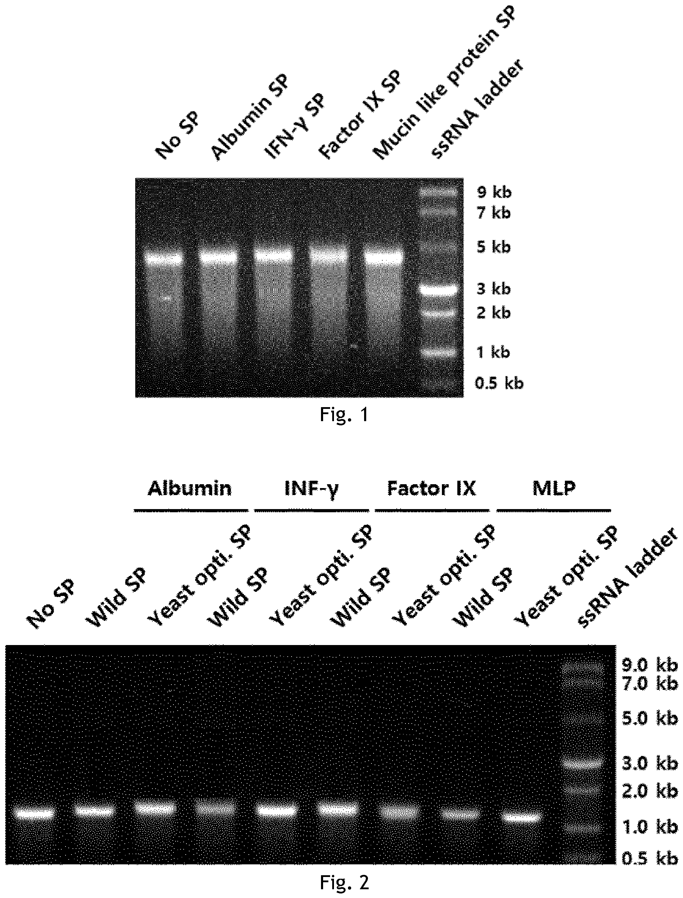

FIG. 1 is an image showing the electrophoresis results of mRNA nucleic acid molecules in which each signal peptide and the SARS-CoV-2 virus spike protein (hereinafter, referred to as 'spike protein') as an antigen protein are fused. `ssRNA ladder' is a size marker for measuring the size of mRNA, `No SP' is the result regarding a nucleic acid molecule containing only the spike protein and no signal peptide, `Albumin SP' is the result regarding a nucleic acid molecule containing albumin-derived signal peptide and spike protein, 'IFN-γ SP' is the result regarding a nucleic acid molecule containing IFN-γ-derived signal peptide and spike protein, `Factor IX SP' is the result regarding a nucleic acid molecule containing Factor IX-derived signal peptide and spike protein, and `Mucin like protein SP' is the result regarding a nucleic acid molecule containing MLP1-derived signal peptide and spike protein.
FIG. 2 is an image showing the electrophoresis results of mRNA nucleic acid molecules in which each signal peptide and the SARS-CoV-2 virus membrane protein (hereinafter, referred to as 'membrane protein') as an antigen protein are fused. `ssRNA ladder' is a size marker for measuring the size of mRNA, `No SP' is the result regarding a nucleic acid molecule containing only the membrane protein and no signal peptide, `Albumin SP' is the result regarding a nucleic acid molecule containing albumin-derived signal peptide and membrane protein, 'IFN-γ SP' is the result regarding a nucleic acid molecule containing IFN-γ-derived signal peptide and membrane protein, `Factor IX SP' is the result regarding a nucleic acid molecule containing Factor IX-derived signal peptide and membrane protein, and 'MLP' is the result regarding a nucleic acid molecule containing MLP1-derived signal peptide and membrane protein. In addition, `Wild SP' is the result using each signal peptide optimized with wild-type codons, and 'Yeast opti. SP' is the result using each signal peptide optimized with yeast codons.

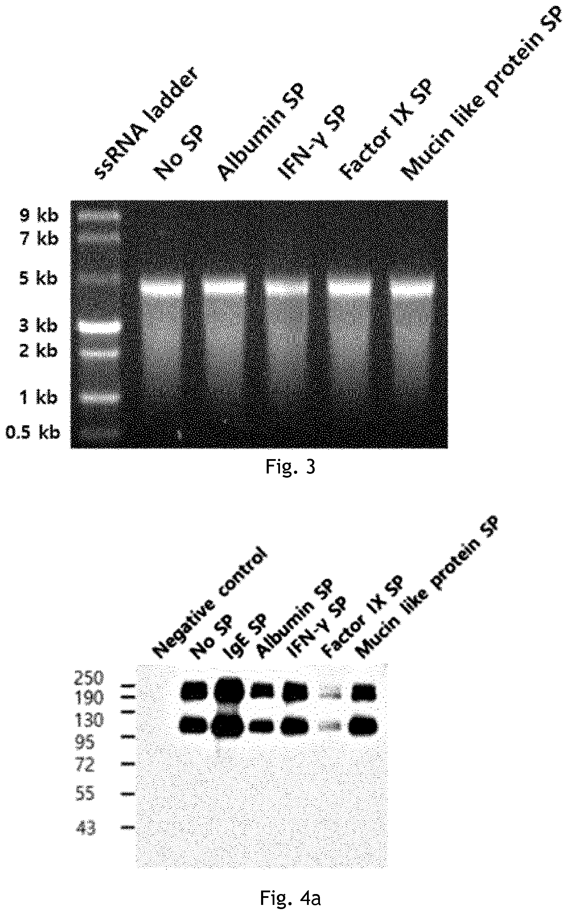

FIG. 3 is an image showing the electrophoresis results of mRNA nucleic acid molecules in which each signal peptide, the SARS-CoV-2 virus spike protein (hereinafter, referred to as 'spike protein') as an antigen protein and Th cell epitope are fused. `ssRNA ladder' is a size marker for measuring the size of mRNA, `No SP' is the result regarding a nucleic acid molecule containing only the spike protein and no signal peptide, `Albumin SP' is the result regarding a nucleic acid molecule containing albumin-derived signal peptide and spike protein, 'IFN-γ SP' is the result regarding a nucleic acid molecule containing IFN-γ-derived signal peptide and spike protein, `Factor IX SP' is the result regarding a nucleic acid molecule containing Factor IX-derived signal peptide and spike protein, and `Mucin like protein SP' is the result regarding a nucleic acid molecule containing MLP1-derived signal peptide and spike protein.
FIG. 4 is an image showing the amount of SARS-CoV-2 virus spike protein (hereinafter, referred to as 'spike protein') expressed in cells, and relates to the amount of protein contained in cell lysates transfected with each nucleic acid molecule. 'Negative control' is the result of a cell not transfected with a nucleic acid molecule, `No SP' is the result regarding a nucleic acid molecule containing only the spike protein and no signal peptide, 'IgE' is the result regarding a nucleic acid molecule containing IgE-derived signal peptide and spike protein, `Albumin SP' is the result regarding a nucleic acid molecule containing albumin-derived signal peptide and spike protein, 'IFN-γ SP' is the result regarding a nucleic acid molecule containing IFN-γ-derived signal peptide and spike protein, `Factor IX SP' is the result regarding a nucleic acid molecule containing Factor IX-derived signal peptide and spike protein, and `Mucin like protein SP' is the result regarding a nucleic acid molecule containing MLP1-derived signal peptide and spike protein.

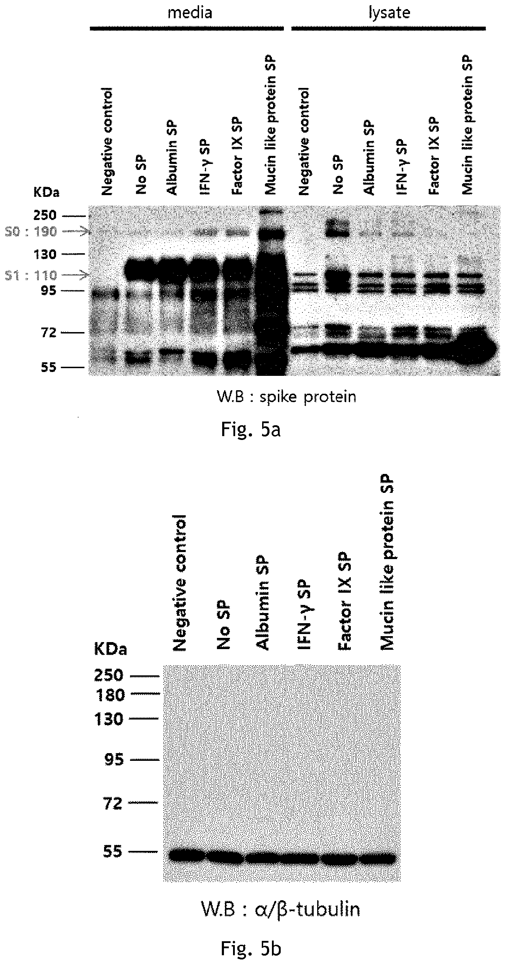

FIG. 5A is an image showing the amount of SARS-CoV-2 virus spike protein (hereinafter, referred to as 'spike protein') expressed in cells, 'media' relates to the amount of protein contained in the culture media of cells transfected with each nucleic acid molecule, and 'lysate' relates to the amount of protein contained in the lysates of cells transfected with each nucleic acid molecule. 'Negative control' is the result of a cell not transfected with a nucleic acid molecule, `No SP' is the result regarding a nucleic acid molecule containing only the spike protein and no signal peptide, `Albumin SP' is the result regarding a nucleic acid molecule containing albumin-derived signal peptide and spike protein, 'IFN-γ SP' is the result regarding a nucleic acid molecule containing IFN-γ-derived signal peptide and spike protein, `Factor IX SP' is the result regarding a nucleic acid molecule containing Factor IX-derived signal peptide and spike protein, and `Mucin like protein SP' is the result regarding a nucleic acid molecule containing MLP1-derived signal peptide and spike protein.
FIG. 5B is an image showing the amount of α/β-tubulin protein used as a loading control, expressed in the cells according to FIG. 5A.

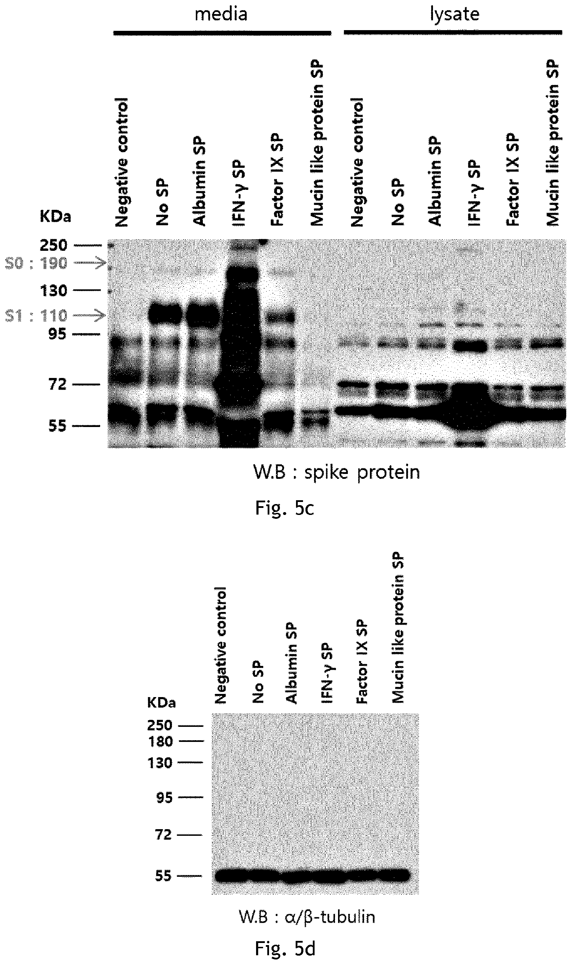

FIG. 5C is an image showing the amount of SARS-CoV-2 virus spike protein (hereinafter, referred to as 'spike protein') expressed in cells, 'media' relates to the amount of protein contained in the culture media of cells transfected with each nucleic acid molecule, and 'lysate' relates to the amount of protein contained in the lysates of cells transfected with each nucleic acid molecule. 'Negative control' is the result of a cell not transfected with a nucleic acid molecule, `No SP' is the result regarding a nucleic acid molecule containing only the spike protein and Th cell epitope, but no signal peptide, `Albumin SP' is the result regarding a nucleic acid molecule containing albumin-derived signal peptide, spike protein and Th cell epitope, 'IFN-γ SP' is the result regarding a nucleic acid molecule containing IFN-γ-derived signal peptide, spike protein and Th cell epitope, `Factor IX SP' is the result regarding a nucleic acid molecule containing Factor IX-derived signal peptide, spike protein and Th cell epitope, and `Mucin like protein SP' is the result regarding a nucleic acid molecule containing MLP1-derived signal peptide, spike protein and Th cell epitope.
FIG. 5D is an image showing the amount of α/β-tubulin protein used as a loading control, expressed in the cells according to FIG. 5C.

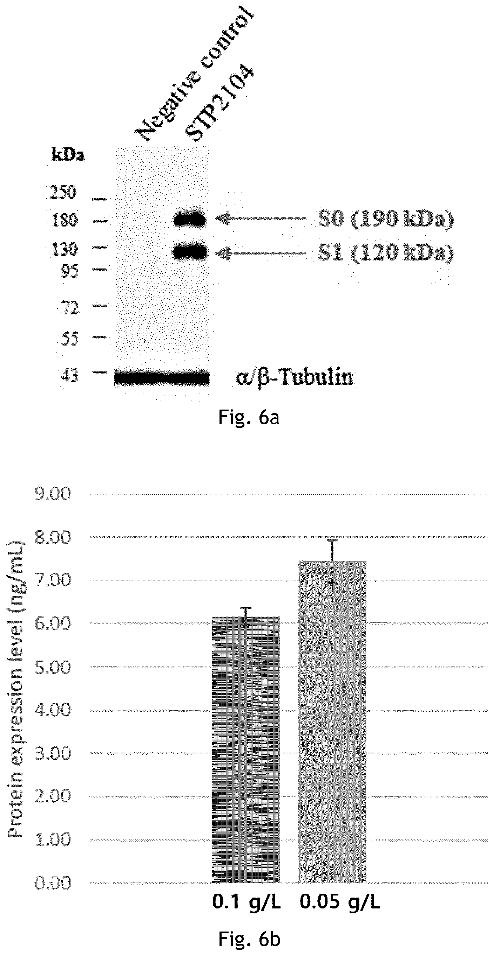

FIG. 6A is an image showing the amount of SARS-CoV-2 virus spike protein expressed in cells, and relates to the results for cells transfected with a nucleic acid molecule containing the IgE-derived signal peptide and the entire SARS-CoV-2 virus spike protein ('STP2104'). 'S0' refers to the entire SARS-CoV-2 virus spike protein, and 'S 1' refers to subunit 1 of the SARS-CoV-2 virus spike protein. 'α/β-tubulin' was used as a loading control.
FIG. 6B is a graph showing the amount of SARS-CoV-2 virus spike protein detected in the culture media of cells, and relates to the results for cells transfected with a nucleic acid molecule containing the IgE-derived signal peptide and the entire SARS-CoV-2 virus spike protein. The nucleic acid molecule was used at a concentration of 0.1 or 0.05 g/L.

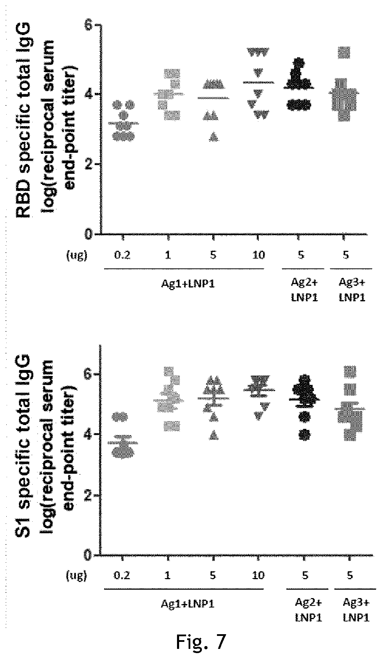

FIG. 7 is a graph showing the amount of SARS-CoV-2 virus spike protein-specific binding antibodies formed in mice, and is the result measured 3 weeks after the second (boost) inoculation. The amount of each antibody was expressed as endpoint titer. 'Ag1+LNP1' is the result regarding a nucleic acid molecule containing the IgE-derived signal peptide and the entire SARS-CoV-2 virus spike protein, and 'Ag2+LNP1' is the result regarding a nucleic acid molecule containing the MLP1-derived signal peptide and the entire SARS-CoV-2 virus spike protein, and 'Ag3+LNP1' is the result regarding the nucleic acid molecule containing the IgE-derived signal peptide, the entire SARS-CoV-2 virus spike protein and the Th cell epitope. In addition, 'RBD specific total IgG' is the result of the amount of antibodies specific to the RBD (receptor binding domain) domain in the SARS-CoV-2 virus spike protein, and 'S1 specific total IgG' is the result of the amount of antibodies specific to the subunit 1 (S 1) in the SARS-CoV-2 virus spike protein.

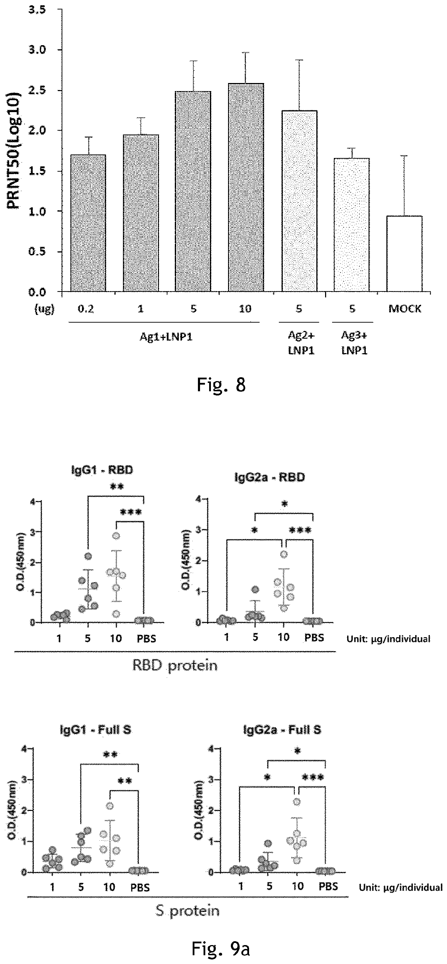

FIG. 8 is a graph showing the amount of SARS-CoV-2 virus spike protein-specific neutralizing antibodies formed in mice, and shows the PRNT (Plaque reduction neutralization test) 50 value converted to Log value. 'Ag1+LNP1' is the result regarding a nucleic acid molecule containing the IgE-derived signal peptide and the entire SARS-CoV-2 virus spike protein, and 'Ag2+LNP1' is the result regarding a nucleic acid molecule containing the MLP1-derived signal peptide and the entire SARS-CoV-2 virus spike protein, 'Ag3+LNP1' is the result regarding the nucleic acid molecule containing the IgE-derived signal peptide, the entire SARS-CoV-2 virus spike protein and the Th cell epitope, and 'Mock' is the result regarding PBS (Phosphate Buffered saline).
FIG. 9A is a graph showing the amount of SARS-CoV-2 virus spike protein-specific binding antibodies (IgG1 and IgG2a isotype) formed in mice, which is the result for mice administered with 1, 5 or 10 µg of nucleic acid molecules containing the IgE-derived signal peptide and the entire SARS-CoV-2 virus spike protein. As a result of measurement at 4 weeks after the first (prime) inoculation, the amount of each antibody was expressed as optical density (O.D.). 'RBD protein' is a result showing that the amount of IgG1 and IgG2a antibodies specific to the RBD (receptor binding domain) domain of the SARS-CoV-2 virus spike protein increases depending on the immunization dose, and 'S protein' is a result showing that the amount of IgG1 and IgG2a antibodies specific for the entire the SARS-CoV-2 virus spike protein increases depending on the immunization dose. The increase in the in vivo IgG1/IgG2a antibody ratio caused by immune injection of the mRNA vaccine means that Th2 type humoral immune response is predominantly induced.

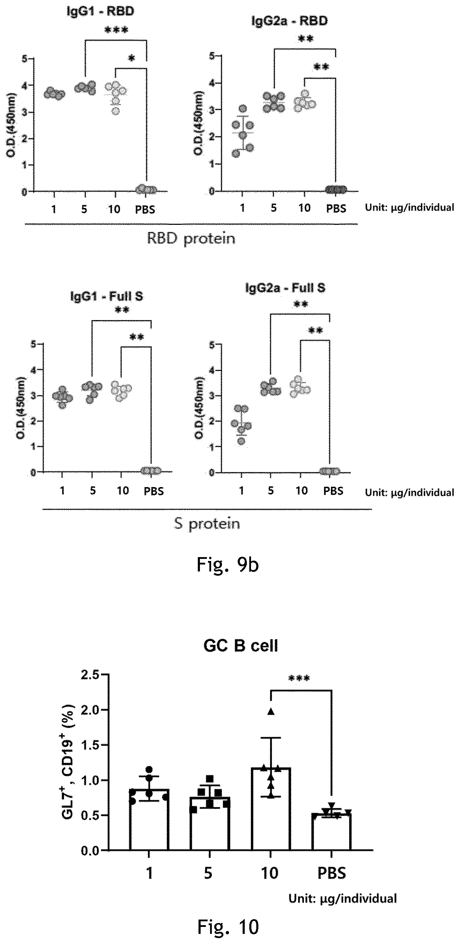

FIG. 9B is a graph showing the amount of SARS-CoV-2 virus spike protein-specific binding antibodies (IgG1 and IgG2a isotype) formed in mice, which is the result for mice administered with 1, 5 or 10 µg of nucleic acid molecules containing the IgE-derived signal peptide and the entire SARS-CoV-2 virus spike protein. As a result of measurement at 3 weeks after the second (boost) inoculation, the amount of each antibody was expressed as optical density (O.D.). 'RBD protein' is a result showing that the amount of IgG1 and IgG2a antibodies specific to the RBD (receptor binding domain) domain of the SARS-CoV-2 virus spike protein increases depending on the immunization dose, and 'S protein' is a result showing that the amount of IgG1 and IgG2a antibodies specific for the entire the SARS-CoV-2 virus spike protein increases depending on the immunization dose. The increase in the in vivo IgG1/IgG2a antibody ratio caused by immune injection of the mRNA vaccine means that Th2 type humoral immune response is predominantly induced.
FIG. 10 is a graph showing the amount of germinal center (GC) B cells formed in mice, in which 1, 5, or 10 µg of nucleic acid molecules containing the IgE-derived signal peptide and the entire SARS-CoV-2 virus spike protein are administered. The humoral immune response requires interactions between specific populations of B cells and CD4+ T cells (follicular helper T cells; T_{FH} cells) in the germinal center (GC) to generate memory B cells and long-lived plasma cells. Molecular crosstalk between GC B cells and T_{FH} cells affects the survival, proliferation, and differentiation of each cell type.

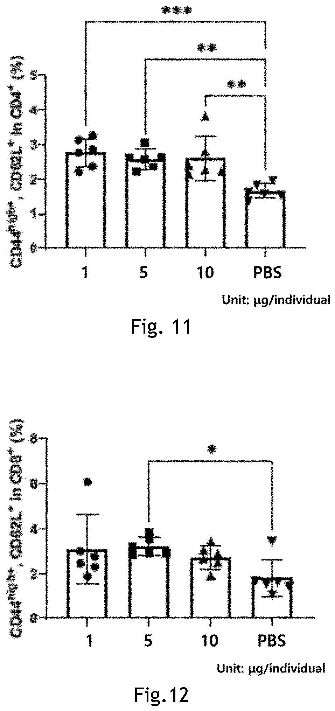

FIG. 11 is a graph showing the amount of CD4⁺ central memory T cells (CD44^{high+} and CD62L⁺ cells among CD4⁺ cells) formed in mice. Splenocytes were isolated from mice administered with 1, 5 or 10 µg of nucleic acid molecules containing the IgE-derived signal peptide and the entire SARS-CoV-2 virus spike protein. A 15-mer long peptide was synthesized with 9-mer overlap for the entire spike amino acid sequence, and then a peptide pool was formed into 5 groups (peptides 1 to 5). The results of flow cytometry analysis of spleen cells stimulated in vitro with peptide pool 2 among them are shown.
FIG. 12 is a graph showing the amount of CD8⁺ central memory T cells (CD44^{high+} and CD62L⁺ cells among CD8⁺ cells) formed in mice. Splenocytes were isolated from mice administered with 1, 5, or 10 µg of nucleic acid molecules containing the IgE-derived signal peptide and the entire SARS-CoV-2 virus spike protein, and then the results obtained by stimulating with peptide pool 2 derived from the SARS-CoV-2 virus spike protein as described above are shown.

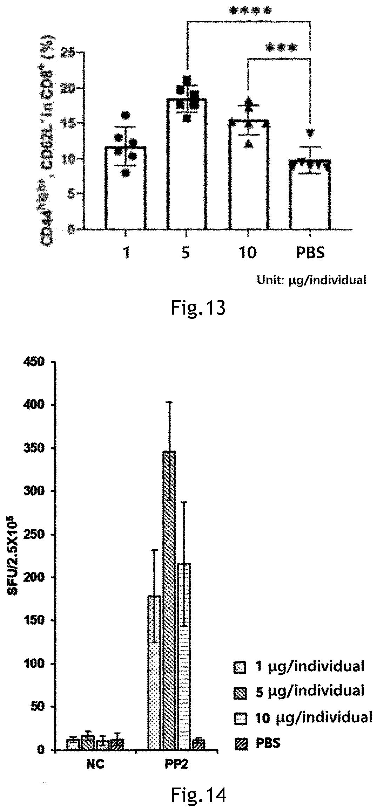

FIG. 13 is a graph showing the amount of CD8⁺ effector memory T cells (CD44^{high+} and CD62L⁻ cells among CD8⁺ cells) formed in mice. Splenocytes were isolated from mice administered 1, 5, or 10 µg of nucleic acid molecules containing the IgE-derived signal peptide and the entire SARS-CoV-2 virus spike protein, and then the results obtained by stimulating with peptide pool 2 derived from the SARS-CoV-2 virus spike protein as described above are shown.
FIG. 14 is a graph showing the results of analyzing the number of IFN-γ secreting cells formed in mice using ELISpot. It shows the results of the number of cells secreting IFN-γ in splenocytes isolated from mice spleen administered with 1, 5, or 10 µg of nucleic acid molecules containing the IgE-derived signal peptide and the entire SARS-CoV-2 virus spike protein. 'NC' is a negative control and shows a result obtained without stimulation with peptide, and 'PP2' shows a result obtained by stimulation with peptide pool 2 derived from the SARS-CoV-2 virus spike protein as described above.

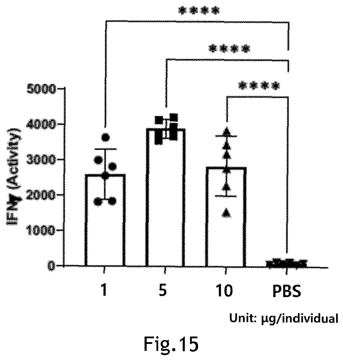

FIG. 15 is a graph showing the degree of IFN-γ staining of the IFN-γ secreting cells formed in mice in terms of activity. Splenocytes were isolated from mice administered with 1, 5, or 10 µg of nucleic acid molecules containing the IgE-derived signal peptide and the entire SARS-CoV-2 virus spike protein, and then the results obtained by stimulating with peptide pool 2 derived from the SARS-CoV-2 virus spike protein as described above are shown.

### [Examples]

The present invention will be explained in more detail based on the following examples, but this is not intended to limit the scope of the present invention. In addition, those skilled in the art will be able to make various changes and modifications to the present invention without impairing the spirit of the present invention.

### Example 1. Preparation of nucleic acid molecules for vaccines

In order to develop a vaccine candidate that can be used to prevent viral infection or cancer, we prepared nucleic acid molecules with excellent cellular immunogenicity and ability to induce neutralizing antibody production, and in particular, with a significantly increased protein expression rate.

Specifically, when the nucleic acid molecule according to the present invention is administered into the body, the expression rate of the antigen protein increases, leading to an enhanced cellular immunogenicity that causes an immune response, and accordingly, a rapid production of a large quantity of neutralizing antibodies in the human body. Furthermore, the immune response by Th cells is efficiently induced and the cytokine secretion ability is increased, thereby showing maximized prevention and/or treatment efficacy against viral infection or cancer.

In order to achieve the above purpose, we comprised signal peptides, Th cell epitopes, etc., and specific antigens for inducing preventive and/or therapeutic efficacy in the nucleic acid molecules. The base and amino acid sequences of the peptides and proteins were obtained from GenBank, and the wild-type sequence obtained from GenBank was optimized with human or yeast codons to prepare nucleic acid molecules with improved antigen protein expression efficiency. Codon optimization involves optimizing the protein-coding gene by modifying it to the codon preferred for each subject so that it can be well expressed. It is carried out taking into account a number of factors, including GC content, repeated base sequence, transcription efficiency, expression level, protein folding, and mRNA secondary structure. Since the correlation between the above factors has various effects on the codon optimization results, there are many cases and it is very difficult to make a mRNA sequence that can show the desired level of expression.

In addition, the protein expression level was predicted by calculating the GC content of the nucleic acid molecule and the free energy of the mRNA secondary structure. As the free energy of the mRNA secondary structure increases, the stability of the structure decreases and the structure easily collapses. As recognition efficiency by ribosomes increases, protein expression rate can be increased.

Nucleic acid molecules were designed using signal peptides, antigens, Th cell epitopes, etc. as follows: 5'-[signal peptide]-[antigen]-3' or 5'-[signal peptide]-[antigen]-[Th cell epitope]-3'.

### Example 1-1. Preparation of signal peptide

In order to prepare nucleic acid molecules for vaccines with excellent protein expression rates, a signal peptide (SP) was first produced.

Specifically, signal peptides derived from five different proteins such as human-derived Immunoglobulin E (IgE), albumin, interferon gamma (IFN-γ), factor IX (F IX), mucin-like protein 1 (MLP1), etc. were obtained. The sequences of these signal peptides were mutated in various ways, and among them, sequences with excellent antigen protein expression were selected. The results are shown in Table 1 below. For example, the nucleic acid sequence of SEQ ID NO: 3 is a codon-optimized form of an IgE-derived signal peptide for human.

**[Table 1]**

| SP type | SP codon | Sequence | SEQ ID NO |
|---|---|---|---|
| IgE | Wild type (amino acid) | MDWTWILFLV AAATRVHS | SEQ ID NO: 1 |
| | Wild type (nucleic acid) | ATGGACTGGACCTGGATCCTCTTCTT GGTGGCAGCAGCCACGCGAGTCCACT CC | SEQ ID NO: 2 |
| | Human | ATGGACTGGACCTGGATCCTGTTCCT GGTGGCCGCCGCTACAAGAGTGCACA GC | SEQ ID NO: 3 |
| | Yeast | ATGGACTGGACTTGGATCTTGTTCTTG GTTGCTGCTGCCACCAGAGTCCACTC T | SEQ ID NO: 4 |
| Albumin | Wild type (amino acid) | MKWVTFISLL FLFSSAYS | SEQ ID NO: 5 |
| | Wild type (nucleic acid) | ATGAAGTGGGTAACCTTTATTTCCCTT CTTTTTCTCTTTAGCTCGGCTTATTCC | SEQ ID NO: 6 |
| | Human | ATGAAGTGGGTGACCTTCATCAGCCT GCTGTTCCTGTTTTCTTCCGCCTACAG C | SEQ ID NO: 7 |
| | Yeast | ATGAAGTGGGTTACTTTCATCTCTTTG TTGTTTTTATTCTCCTCTGCTTACTCC | SEQ ID NO: 8 |
| IFN-γ | Wild type (amino acid) | MKYTSYILAF QLCIVLGSLG CYC | SEQ ID NO: 9 |
| | Wild type (nucleic acid) | ATGAAATATACAAGTTATATCTTGGC TTTTCAGCTCTGCATCGTTTTGGGTTC TCTTGGCTGTTACTGC | SEQ ID NO: 10 |
| | Human | ATGAAGTACACCAGCTACATCCTGGC CTTCCAGCTGTGCATCGTGCTGGGCT CTCTGGGCTGTTACTGC | SEQ ID NO: 11 |
| | Yeast | ATGAAGTACACTTCTTACATTTTGGCT TTCCAATTGTGTATCGTTTTGGGTTCC TTAGGTTGTTACTGT | SEQ ID NO: 12 |
| FIX | Wild type (amino acid) | MQRVNMIMAE SPGLITICLL GYLLSAEC | SEQ ID NO: 13 |
| | Wild type (nucleic acid) | ATGCAGCGCGTGAACATGATCATGGC AGAATCACCAGGCCTCATCACCATCT GCCTTTTAGGATATCTACTCAGTGCTG AATGT | SEQ ID NO: 14 |
| | Human | ATGCAGAGAGTGAACATGATCATGGC CGAGTCTCCTGGCCTGATCACCATCT GCCTGCTGGGCTACCTGCTGAGCGCC GAATGT | SEQ ID NO: 15 |
| | Yeast | ATGCAAAGAGTTAACATGATTATGGC TGAATCTCCAGGTTTGATCACTATCTG TTTGTTGGGTTACTTGTTATCCGCCGA ATGT | SEQ ID NO: 16 |
| MLP1 | Wild type (amino acid) | MVYKTLFALC ILTAGWRVQS | SEQ ID NO: 17 |
| | Wild type (nucleic acid) | ATGGTCTACAAGACTCTCTTCGCTCTT TGCATCTTAACTGCAGGATGGAGGGT ACAGAGT | SEQ ID NO: 18 |
| | Human | ATGGTGTACAAGACCCTGTTCGCCCT GTGCATCCTGACCGCCGGCTGGAGGG TGCAGAGC | SEQ ID NO: 19 |
| | Yeast | ATGGTATATAAAACTTTATTTGCTTTA TGTATTTTAACTGCTGGTTGGCGTGTA CAATCT | SEQ ID NO: 20 |

### Example 1-2. Antigen preparation

### Example 1-2-1. Preparation of 'SARS-CoV-2' spike protein

In order to develop a vaccine candidate that can be used to prevent viral infection or cancer, we used as an example the antigen of 'SARS-CoV-2', the virus that causes COVID-19.

Specifically, spike protein (S), which is known to play a central role in infection and pathogenicity such as receptor recognition of host cell, cell membrane fusion, and induction of neutralizing antibody, was used. Then, codon optimization was performed using the method according to Example 1-1, and the results are shown in Table 2 below. For example, the nucleic acid sequence of SEQ ID NO: 23 is a codon-optimized form of the spike protein antigen of SARS-CoV-2 for humans, and the nucleic acid sequence of SEQ ID NO: 25 is a codon-optimized form of the spike protein antigen of SARS-CoV-2 for both humans and yeast.

**[Table 2]**

| Antigen type | Antigen codon | SEQ ID NO |
|---|---|---|
| SARS-CoV-2 spike protein | Wild type (amino acid) | SEQ ID NO: 21 |
| | Wild type (nucleic acid) | SEQ ID NO: 22 |
| | Human | SEQ ID NO: 23 |
| | Yeast | SEQ ID NO: 24 |
| | Yeast codon 250 nt and human codon 3569 nt | SEQ ID NO: 25 |

As a result, it was confirmed that the form having both yeast codons and human codons have a high expression rate of viral antigens in human cells and also exhibit the desired infection prevention effect to an excellent degree.

Therefore, the nucleic acid of SEQ ID NO: 25, which has both yeast codons and human codons, was used as the antigen for the subsequent preparation of nucleic acid molecules for vaccines.

### Example 1-2-2. Preparation of 'SARS-CoV-2' membrane protein

In order to develop a vaccine candidate that can be used to prevent viral infection or cancer, we used as an example the antigen of 'SARS-CoV-2', the virus that causes COVID-19.

Specifically, when comparing genes between various variants of coronavirus, membrane protein (M), a well-conserved gene, was used. Then, codon optimization was performed using the method according to Example 1-1, and the results are shown in Table 3 below. For example, the nucleic acid sequence of SEQ ID NO: 28 is a codon-optimized form of the membrane protein antigen of SARS-CoV-2 for humans, and the nucleic acid sequence of SEQ ID NO: 30 is a codon-optimized form of the membrane protein antigen of SARS-CoV-2 for both humans and yeast.

**[Table 3]**

| Antigen type | Antigen codon | SEQ ID NO |
|---|---|---|
| SARS-CoV-2 membrane protein | Wild type (amino acid) | SEQ ID NO: 26 |
| | Wild type (nucleic acid) | SEQ ID NO: 27 |
| | Human | SEQ ID NO: 28 |
| | Yeast | SEQ ID NO: 29 |
| | Yeast codon 250 nt and human codon 419 nt | SEQ ID NO: 30 |

As a result, it was confirmed that the form having both yeast codons and human codons have a high expression rate of antigens in human cells and also exhibit the desired infection prevention effect to an excellent degree.

Therefore, the nucleic acid of SEQ ID NO: 30, which has both yeast codons and human codons, was used as the antigen for the subsequent preparation of nucleic acid molecules for vaccines.

### Example 1-3. Fusion of signal peptide and antigen

### Example 1-3-1. Use of 'SARS-CoV-2' spike protein as an antigen

In order to prepare nucleic acid molecules with increased protein expression efficiency, nucleic acid molecules were prepared by fusing the five types of signal peptides obtained through Example 1-1 and the spike proteins of the SARS-CoV-2 virus obtained through Example 1-2-1.

Specifically, the spike protein used was a form having both yeast codons and human codons, and summarized in Table 4 below.

**[Table 4]**

| SP type | SP codon | SARS-CoV-2 spike protein | SEQ ID NO |
|---|---|---|---|
| IgE | Yeast | Yeast codon 250 nt and human codon 3569 nt | SEQ ID NO: 31 |
| Albumin | Yeast | | SEQ ID NO: 32 |
| IFN-γ | Yeast | | SEQ ID NO: 33 |
| FIX | Yeast | | SEQ ID NO: 34 |
| MLP1 | Yeast | | SEQ ID NO: 35 |

Then, in order to synthesize the nucleic acid molecule to mRNA, we mixed 10X T7 RNA polymerase buffer (Dynebio, catalog # DYP1647), 2mM ATP, 2mM UTP, 2mM CTP, 2mM GTP, 3.2mM Cleancap AG (Trilink, catalog #N-7113-10), 5% DMSO (Sigma Aldrich Catalog #472301-500ML), 10 mg/L antigen (SARS-CoV-2 spike protein) DNA transcription template, 800 U/mL recombinant RNase inhibitory protein (Takara Catalog #2316A), 2 U/mL yeast inorganic pyrophosphatase (Thermo Scientific catalog #EF0221), and 2500 U/mL T7 RNA polymerase (DyneBio catalog #dy1670). The transcription reaction mixture was incubated at 37°C for 2 hours. The synthesis of mRNA was confirmed by electrophoresis using a 1% agarose gel supplemented with Lonza GelStar^{™} Nucleic Acid gel Stain (Catalog #5 0535). The synthesized mRNA was purified by AKTA (Cytiva, AKTA avant) using an Oligo (dT) column (Via Separations, catalog # 311.1219-2) and then used in subsequent in vitro or in vivo experiments.

As a result, as can be seen in FIG. 1, it was confirmed that all nucleic acid molecules in which each signal peptide and the antigen protein were fused showed a band with a size of approximately 4,119 nt. Since this indicates a size including both signal peptides and antigen proteins, it was confirmed that the mRNA nucleic acid molecule for vaccine synthesized in this example was well manufactured and its integrity was secured.

### Example 1-3-2. Use of 'SARS-CoV-2' membrane protein as an antigen

In order to prepare nucleic acid molecules with increased protein expression efficiency, nucleic acid molecules were prepared by fusing the four types of signal peptides obtained through Example 1-1 and the membrane proteins of the SARS-CoV-2 virus obtained through Example 1-2-2, and these are summarized in Table 5 below.

**[Table 5]**

| SP type | SP codon | SARS-CoV-2 membrane protein | SEQ ID NO |
|---|---|---|---|
| Albumin | Wild type | Yeast codon 250 nt and human codon 419 nt | SEQ ID NO: 36 |
| | Yeast | | SEQ ID NO: 37 |
| IFN-γ | Wild type | | SEQ ID NO: 38 |
| | Yeast | | SEQ ID NO: 39 |
| FIX | Wild type | | SEQ ID NO: 40 |
| | Yeast | | SEQ ID NO: 41 |
| MLP1 | Wild type | | SEQ ID NO: 42 |
| | Yeast | | SEQ ID NO: 43 |

Afterwards, the nucleic acid molecule was synthesized to mRNA by the method according to Example 1-3-1, and then the synthesis was confirmed, and the synthesized mRNA was purified.

As a result, as can be seen in FIG. 2, it was confirmed that all nucleic acid molecules in which each signal peptide and the antigen protein were fused showed a band with a size of approximately 1,107 nt. Since this indicates a size including both signal peptides and antigen proteins, it was confirmed that the mRNA nucleic acid molecule for vaccine synthesized in this example was well manufactured and its integrity was secured.

### Example 1-4. Preparation of Th cell epitope

In order to prepare a nucleic acid molecule for a vaccine with increased cellular immunogenicity and ability to induce an immune response, a Th cell epitope was first prepared.

Specifically, we used as an example the Th cell epitope derived from tetanus toxoid (Tetanus Toxoid Th cell epitope, TTTh). In addition, the TTTh was linked to a cleavage site (CS) with different sequences. The Th cell epitope containing the cleavage site was designed to include a termination codon as follows and was located at the C-terminus of the ORF: 5'-[CS1]-[TTTh1]-[CS2]-[TTTh2]- [termination codon]-3'. The specific amino acid sequence corresponding to the above construct is as follows: 5'-[RQKR]-[IDKISDVSTIVPYIGPALNI]-[PKKR]-[NNFTVSFWLRVPKVSASHLE]-3'. Afterwards, codon optimization was performed using the method according to Example 1-1, and the results are summarized in Table 6 below. For example, the nucleic acid sequence of SEQ ID NO: 46 is a codon-optimized form of TTTh for humans.

**[Table 6]**

| | | | |
|---|---|---|---|
| Th cell epitope | Th cell epitope codon | Sequence | SEQ ID NO |
| | Wild type (amino acid) | RQKRIDKISD VSTIVPYIGP ALNIPKKRNN FTVSFWLRVP KVSASHLE | SEQ ID NO: 44 |
| | Wild type (nucleic acid) | CGCCAGAAACGCATTGATAAAATTAGC GATGTGAGCACCATTGTGCCGTATATT GGCCCGGCGCTGAACATTCCGAAAAAA CGCAACAACTTTACCGTGAGCTTTTGGC TGCGCGTGCCGAAAGTGAGCGCGAGCC ATCTGGAATGA | SEQ ID NO: 45 |
| | Human | AGACAGAAAAGAATCGATAAGATCAG CGACGTGTCTACAATCGTGCCATACAT CGGCCCTGCCCTGAATATCCCCAAGAA GCGGAACAACTTCACCGTCAGCTTCTG GCTGAGAGTGCCTAAGGTGTCCGCCAG CCACCTGGAGTGA | SEQ ID NO: 46 |

As a result, it was confirmed that the form with human codons have a high Th cell epitope expression rate in human cells and also exhibit the desired vaccine effect to an excellent degree.

### Example 1-5. Fusion of signal peptide, antigen and Th cell epitope

### Example 1-5-1. Use of 'SARS-CoV-2' spike protein as an antigen

Through Examples 1-1 to 1-3, etc., it was confirmed that the signal peptide proven to have a high protein expression rate is the form with a yeast codon, and the antigen 'SARS-CoV-2' spike protein proven to have a high protein expression rate is the form with both yeast and human codons. A nucleic acid molecule was prepared by fusing the Th cell epitope sequence to the signal peptide sequence and antigen sequence identified in this way. The sequences are summarized in Table 7 below.

**[Table 7]**

| SP type | SP codon | Antigen | Th cell epitope | SEQ ID NO |
|---|---|---|---|---|
| IgE | Yeast codon | SARS-CoV-2 spike protein (Yeast codon 250 nt and human codon 3569 nt) | Human codon | SEQ ID NO: 47 |
| Albumin | | | | SEQ ID NO: 48 |
| IFN-γ | | | | SEQ ID NO: 49 |
| F IX | | | | SEQ ID NO: 50 |
| MLP1 | | | | SEQ ID NO: 51 |

Thereafter, the nucleic acid molecules were synthesized to mRNA by the method according to Example 1-3-1, the synthesis of mRNA was confirmed, and the synthesized mRNA was purified.

As a result, as can be seen in FIG. 3, it was confirmed that all nucleic acid molecules in which each signal peptide and the antigen protein were fused show a band with a size of approximately 4,119 nt. Since this is a size that includes each signal peptide, antigen protein, and Th cell epitope, it was confirmed that the mRNA nucleic acid molecule for vaccine synthesized in this example was well manufactured and its integrity was secured.

### Example 1-5-2. Use of 'SARS-CoV-2' membrane protein as an antigen

Through Examples 1-1 to 1-3, etc., it was confirmed that the signal peptide proven to have a high protein expression rate is the form with a wild-type codon or a yeast codon, and the antigen 'SARS-CoV' membrane protein proven to have a high protein expression rate is the form with both yeast and human codons. Nucleic acid molecules were prepared by fusing the Th cell epitope sequence to the signal peptide sequence and antigen sequence identified in this way. The sequences are summarized in Table 8 below.

**[Table 8]**

| SP type | SP codon | Antigen | Th cell epitope | SEQ ID NO |
|---|---|---|---|---|
| albumin | Wild type | SARS-CoV-2 membrane protein (Yeast codon 250 nt and human codon 419 nt) | Human codon | SEQ ID NO: 52 |
| | Yeast | | | SEQ ID NO: 53 |
| IFN-γ | Wild type | | | SEQ ID NO: 54 |
| | Yeast | | | SEQ ID NO: 55 |
| FIX | Wild type | | | SEQ ID NO: 56 |
| | Yeast | | | SEQ ID NO: 57 |
| MLP1 | Wild type | | | SEQ ID NO: 58 |
| | Yeast | | | SEQ ID NO: 59 |

### Example 2. Effect of nucleic acid molecules for vaccines containing signal peptides

### Example 2-1. Analysis of protein expression level of nucleic acid molecules for vaccines

In order to confirm the efficacy of the nucleic acid molecule prepared in Example 1, the protein expression rate was analyzed in vitro. It is generally known that if the expression characteristics are high in vitro, the vaccine exhibits high immunogenicity in vivo, thereby increasing the efficacy of the vaccine. Among the nucleic acid molecules prepared in Example 1, the effect on the nucleic acid molecules prepared in Examples 1-3-1 or 1-5-1 was confirmed. The entire SARS-CoV-2 virus spike protein (SEQ ID NO: 21), which has both yeast codons and human codons, was used as the antigen. As the signal peptides, those derived from IgE (SEQ ID NO: 4), albumin (SEQ ID NO: 8), interferon gamma (SEQ ID NO: 12), factor IX (SEQ ID NO: 16), or MLP1 (SEQ ID NO: 20) which were all optimized with yeast codons. And, the Th cell epitope optimized with human codon (SEQ ID NO: 46) was used.

Specifically, HEK-293T (ATCC, catalog number CRL-1586) cells were cultured in DMEM medium containing 10% FBS and 1% Pen/Strep, and 4 × 10⁵ cells/well of HEK-293T cells were transfected by the nucleic acid molecules prepared according to the above examples at a concentration of about 0.1 or 0.05 g/L. Herein, the nucleic acid molecules were formulated in the form of mRNA-LNP, where they were encapsulated within lipid nanoparticles (LNPs) as a delivery vehicle. After transfection was completed, the intracellular protein expression of the nucleic acid molecule and the resulting protein secretion out of the cell were analyzed through Western blot and ELISA according to methods known in the art. Western blot was performed using SARS-CoV-2 Spike RBD polyclonal antibody (E-AB-V1006, Elabscience) as the primary antibody against the lysate of the transfected cells, and HRP-conjugated secondary antibody (goat anti-rabbit IgG; ABclonal) as the secondary antibody. ELISA was performed on the culture media of the transfected cells using the SARS-CoV-2 Spike S1 protein ELISA kit (RK04154, ABclonal).

Meanwhile, the spike protein of SARS-CoV-2 used as an antigen is composed of subunit 1 (S1) and subunit 2 (S2), and the receptor binding domain (RBD), which is known to play a major role in cell infection, is located in S 1. The primary antibody used in Western blot targets the RBD domain of the spike protein, and thus the expression and secretion level of the spike protein of SARS-CoV-2 were analyzed by confirming whether the entire spike protein (S0, 190 kDa) is detected, or subunit 1 (S1, 120 kDa), where the RBD domain of the spike protein is located, is detected.

As a result, as can be seen in FIG. 4, it was confirmed that the nucleic acid molecules containing the SARS-CoV-2 virus spike proteins according to the present invention and the signal peptides derived from IgE, albumin, IFN-γ, factor IX or MLP1 are well expressed to proteins within the cells.

In addition, as can be seen in FIGs. 5A and 5B, it was confirmed that the nucleic acid molecules containing the SARS-CoV-2 viral spike proteins according to the present invention and the signal peptides derived from albumin, IFN-γ, factor IX or MLP1 are well expressed to proteins within the cells and are smoothly secreted outside the cells.

In addition, as can be seen in in FIGs. 5C and 5D, it was confirmed that the nucleic acid molecules containing SARS-CoV-2 viral spike proteins according to the present invention, signal peptides derived from albumin, IFN-γ, factor IX or MLP1, and Th cell epitopes are well expressed to proteins within the cells and are smoothly secreted outside the cells.

In particular, in the case of nucleic acid molecules containing the signal peptide, it was confirmed that the amount of protein contained in the culture media was greater than that contained in the cell lysate. In addition, when comparing the protein expression levels of the nucleic acid molecule containing the signal peptide and the nucleic acid molecule not containing it, the protein amount of the nucleic acid molecule containing the signal peptide was greater in the culture media, and the protein amount of nucleic acid molecules not containing the signal peptide was greater in the cell lysate. Through this, it was confirmed that extracellular secretion of spike protein can be promoted by the signal peptide.

In addition, as can be seen in FIG. 6A, it was confirmed that the nucleic acid molecule containing SARS-CoV-2 virus spike protein and IgE-derived signal peptide according to the present invention is well expressed even within the cells.

In addition, as can be seen in FIG. 6B, it was confirmed that the nucleic acid molecule containing the SARS-CoV-2 virus spike protein according to the present invention is well expressed within the cells, and accordingly a significant amount of the protein is secreted outside the cell, as shown by spike protein ELISA. For mRNA-LNP stock samples of different concentrations, approximately 6.32 ng/mL of protein was expressed and secreted in cells transfected with 0.1 g/L sample, and approximately 7.71 ng/mL in cells transfected with 0.05 g/L sample. It was confirmed that a similar amount of spike antigen protein was expressed and secreted.

The above results show that the nucleic acid molecule according to the present invention, which is composed of mRNA-LNP formulation and contains each signal peptide and SARS-CoV-2 virus spike protein as an antigen, is expressed as a protein with high efficiency within cells, in particular, the expressed proteins can be secreted outside the cell, and accordingly this secreted antigen protein can stimulate other immune cells present in the body and induce a cellular or humoral immune response. Therefore, the nucleic acid molecule according to the present invention can exert the desired vaccine function when administered in vivo.

### Example 2-2. Humoral immunity enhancing effect of nucleic acid molecules for vaccine

In order to confirm the immunological efficacy of the nucleic acid molecule prepared in Example 1, it was observed whether the nucleic acid molecule, when administered in vivo, occurs a humoral immune response to the antigen and has a preventive effect against actual viral infection. Among the nucleic acid molecules prepared in Example 1, the effect was confirmed for the nucleic acid molecules prepared in Example 1-3-1 or Example 1-5-1. The entire SARS-CoV-2 virus spike protein (SEQ ID NO: 21), which has both yeast codons and human codons, was used as the antigen. The signal peptide derived from IgE (SEQ ID NO: 4) or MLP1 (SEQ ID NO: 20), optimized with yeast codons was used. And, the Th cell epitope optimized with human codons (SEQ ID NO: 46) was used.

Specifically, the nucleic acid molecule was formulated into mRNA-LNP, and was injected into the upper thigh muscle in 6-week-old female BALB/c mice (6 mice) twice at 4-week intervals (1st: prime, 2nd: boost). They were sacrificed 3 weeks after injection. The nucleic acid molecule was injected at a concentration of 1, 5, or 10 µg/individual. As a control group, PBS was injected.

Thereafter, the level of specific binding antibodies or neutralizing antibodies against the SARS-CoV-2 virus spike protein as an antigen in the serum of the immunized mice was measured through ELISA or Plaque Reduction Neutralizing Test (PRNT) assay. As the primary antibody for ELISA, anti-mouse IgG1-antibody (Invitrogen, Carlsbad, CA, USA) or anti-mouse IgG2a-antibody (Novus, Centennial, CO, USA) was used.

In addition, spleen cells were isolated from the immunized mice, and were treated with a peptide derived from the SARS-CoV-2 virus spike protein at a concentration of 2 mg/ml and cultured for 24 hours. After culture was completed, immunostaining with each specific antibody was performed, and flow cytometry was performed.

As a result, as can be seen in FIGs. 7 to 9B, it was confirmed that binding antibodies against RBD or the entire spike protein were produced at a high level in mice after inoculation with the nucleic acid molecule. In addition, it was confirmed that the amount of binding antibodies and neutralizing antibodies produced increases in a concentration-dependent manner with respect to the nucleic acid molecule (FIGs. 7 to 9B), and the amount of neutralizing antibodies produced after the second inoculation increases by about two-fold compared to that of the first inoculation (FIGs. 9A and 9B).

In addition, as can be seen in FIG. 10, it was confirmed that there was a significant increase in the number of B cells (GC B cells, GL7+ CD19+ cells) present in the germinal center (GC) when the nucleic acid molecule is inoculated into mice at a concentration of 10 µg/individual, compared to non-vaccinated mice. In the germinal center (GC), B cells interact with CD4+ T cells called follicular helper T cells (T_{FH} cells) to generate memory B cells and long-lived plasma cells. These interactions between GC B cells and T_{FH} cells affect the survival, proliferation, and differentiation of each cell type.

The above results show that the nucleic acid molecule composed of mRNA-LNP agent can efficiently induce a humoral immune response to the SARS-CoV-2 virus spike protein, and it suggests that it can be useful for preventing, improving or treating SARS-CoV-2 virus infection.

### Example 2-3. Cellular immunity enhancing effect of nucleic acid molecules for vaccine

### Example 2-3-1. Increased T cells

In order to confirm the immunological efficacy of the nucleic acid molecule prepared in Example 1, it was observed whether the nucleic acid molecule, when administered in vivo, occurs a cellular immune response to the antigen and has a preventive effect against actual viral infection. As a type of cellular immune response, it was confirmed whether the production of T cells such as effector memory T cells and central memory T cells could be increased. The effector memory T cells provide immediate but not lasting defense at the site of pathogen entry, while the central memory T cells maintain the immune response by proliferating in secondary lymphoid organs to generate new effector cells.

In addition, among the nucleic acid molecules prepared in Example 1, the effect on the nucleic acid molecule prepared in Example 1-3-1 was confirmed. The entire SARS-CoV-2 virus spike protein (SEQ ID NO: 21), which has both yeast codons and human codons, was used as the antigen. The signal peptide optimized with yeast codons and derived from IgE (SEQ ID NO: 4) was used. The effect on nucleic acid molecules prepared using this method was confirmed.

Specifically, by the method according to Examples 2-2 and 2-3, the nucleic acid molecule according to the present invention composed of mRNA-LNP agent was injected into mice, spleen cells were isolated from the immunized mice, and then a peptide derived from the SARS-CoV-2 viral spike protein (peptide pool 2) was treated at a concentration of 2 mg/ml. And then, flow cytometry was performed. Proportion of CD4⁺ central memory T cells (CD44^{high+} and CD62L⁺ cells among CD4⁺ cells), CD8⁺ central memory T cells (CD44^{high+} and CD62L⁺ cells among CD8⁺ cells), and CD8⁺ effector memory T cells (CD44^{high+} and CD62L⁻cells among CD8⁺ cells) was analyzed.

As a result, as can be seen in FIGs. 11 to 13, it was confirmed that when the nucleic acid molecule is inoculated into mice, the ratio of cell population contributing to cellular immunity, such as CD4+ central memory T cells, CD8+ central memory T cells, and CD8+ effector memory T cells, is significantly increased compared to non-vaccinated mice.

The above results show that the nucleic acid molecule composed of mRNA-LNP agent can efficiently induce a cellular immune response to the SARS-CoV-2 virus spike protein, thereby preventing, improving or treating SARS-CoV-2 virus infection.

### Example 2-3-2. Increased IFN-γ secreting cells

In order to confirm the efficacy of the nucleic acid molecule prepared in Example 1, it was observed whether it could increase the production of cells secreting IFN-γ as a type of cellular immune response. IFN-γ is an important component responsible for the innate antiviral response and is mainly produced by NK cells or innate lymphocyte type 1 cells. If the production of IFN-γ by these immune cells is not achieved, viral replication increases in vivo.

In addition, among the nucleic acid molecules prepared in Example 1, the effect on the nucleic acid molecules prepared in Example 1-3-1 or Example 1-5-1 was confirmed. The entire SARS-CoV-2 virus spike protein (SEQ ID NO: 21), which has both yeast codons and human codons, was used as the antigen. The signal peptide optimized with yeast codons and derived from IgE (SEQ ID NO: 4) or MLP1 (SEQ ID NO: 20) was used. And, the Th cell epitope optimized with human codons (SEQ ID NO: 46) was used.

Specifically, spleen cells were isolated from mice immunized according to Example 2-2, were treated with SARS-CoV-2 virus spike protein-derived peptide (peptide pool 2) at a concentration of 2 mg/ml, and cultured for 48 hours. After culture was completed, cells secreting IFN-γ were detected using the ELISpot basic kit (Mabtech, Nacka Strand, Sweden).

As a result, as can be seen in FIGs. 14 and 15, it was confirmed that when the nucleic acid molecule was inoculated into mice, the number of cells secreting IFN-γ is significantly increased compared to mice that were not inoculated (FIG. 14). In addition, it was confirmed that the activity of IFN-γ by the cells was significantly increased by about 3000-fold or more (FIG. 15).

The above results show that the nucleic acid molecule composed of mRNA-LNP agent can efficiently induce a cellular immune response to the SARS-CoV-2 virus spike protein, thereby preventing, improving or treating the SARS-CoV-2 virus infection.

### Example 2-3-3. Plaque reduction neutralization titer (PRNT) assay

In order to confirm the efficacy of the nucleic acid molecule prepared in Example 1, it was observed whether it could increase the production of neutralizing antibodies by memory T cells as a type of cellular immune response. The plaque reduction neutralizing titer (PRNT) assay is a widely accepted approach to measure the viral neutralizing and protective antibodies against viruses and to evaluate the immunogenicity of vaccines.

In addition, among the nucleic acid molecules prepared in Example 1, the effect on the nucleic acid molecule prepared in Example 1-3-1 was confirmed. The entire SARS-CoV-2 virus spike protein (SEQ ID NO: 21), which has both yeast codons and human codons, was used as the antigen. The signal peptide optimized with yeast codons and derived from IgE (SEQ ID NO: 4) was used. The effect on nucleic acid molecules prepared using this method was confirmed.

Specifically, SARS-CoV-2 virus (NCCP 43326, S-type) at a concentration of 4.5×10² PFU/ml was added to the serum isolated from mice immunized according to Example 2-2 and cultured for 1 hour. Thereafter, the serum-virus culture was added to Vero E6 cells (ATCC, catalog number CRL-1586) and incubated for 1 hour to infect the cells with the virus. Then, a PRNT analysis assay was performed according to a method known in the art. PRNT titers were calculated using the Karber method and expressed as neutralizing dose ND₅₀ (log₁₀ND₅₀ = m - Δ(Σ-0.5), where m represents the log₁₀ of the highest dilution, Δ represents the log₁₀ of the dilution factor, and ∑ represents the sum of the average number of plaques produced by virus-serum inoculum/the average number of plaques produced by virus controls).

**[Table 9]**

| Dose | Titer after 1st inoculation | Titer after 2nd inoculation |
|---|---|---|
| 1 µg/individual | <10 | 20 |
| | 10 | 20 |
| | 10 | 80 |
| | <10 | 80 |
| | <10 | 20 |
| | 10 | 80 |
| 5 µg/individual | <10 | >2560 |
| | 10 | 80 |
| | 10 | >2560 |
| | 10 | >2560 |
| | 10 | >2560 |
| | 10 | >2560 |
| 10 µg/individual | 10 | >2560 |
| | 10 | 1280 |
| | 10 | 640 |
| | <40 | >2560 |
| | 10 | 1280 |
| | 160 | >2560 |
| PBS | 10 | 10 |
| | <10 | 10 |
| | 20 | 10 |
| | 10 | <10 |
| | <20 | 10 |
| | 10 | 10 |

As a result, as can be seen in Table 9, when the nucleic acid molecule is inoculated into mice, the amount of neutralizing antibodies produced was similar to that of the control group after the first inoculation. However, after the second inoculation, the amount of neutralizing antibodies produced was significantly higher. This effect was confirmed to be particularly excellent when 5 or 10 µg per subject was administered.

The above results show that the nucleic acid molecule composed of mRNA-LNP agent can efficiently generate neutralizing antibodies against the SARS-CoV-2 virus spike protein, thereby preventing, improving or treating SARS-CoV-2 virus infection.

## Claims

1. A nucleic acid molecule for preventing or treating viral infection or cancer, comprising a nucleic acid encoding a signal peptide derived from one or more selected from the group consisting of Immunoglobulin E (IgE), albumin, interferon gamma (IFN-γ), factor IX and mucin-like protein 1 (MLP1), and a nucleic acid encoding an antigen.

2. The nucleic acid molecule according to claim 1, wherein the nucleic acid encoding the signal peptide is one or more selected from the group consisting of SEQ ID NOs: 2 to 4, 6 to 8, 10 to 12, 14 to 16, and 18 to 20.

3. The nucleic acid molecule according to claim 1, wherein the antigen is derived from one or more selected from the group consisting of coronavirus, oncovirus, tumor-specific antigen, tumor-associated antigen and neoantigen epitope.

4. The nucleic acid molecule according to claim 1, wherein the antigen is one or more selected from the group consisting of spike protein and membrane protein derived from coronavirus.

5. The nucleic acid molecule according to claim 1, wherein the nucleic acid encoding the antigen is one or more selected from the group consisting of SEQ ID NOs: 22 to 25, and 27 to 30.

6. The nucleic acid molecule according to claim 1, wherein the nucleic acid molecule comprises a nucleic acid encoding Th cell epitope.

7. The nucleic acid molecule according to claim 6, wherein the Th cell epitope is derived from one or more selected from the group consisting of Tetanus toxoid, diphtheria toxoid (DTH toxoid) and Purtussis toxoid.

8. The nucleic acid molecule according to claim 6, wherein the nucleic acid encoding Th cell epitope is one or more selected from the group consisting of SEQ ID NOs: 45 and 46.

9. The nucleic acid molecule according to claim 1, wherein the viral infection is a coronavirus infection.

10. The nucleic acid molecule according to claim 9, wherein the coronavirus infection is one or more selected from the group consisting of Severe Acute Respiratory Syndrome (SARS), Middle East Respiratory Syndrome (MERS) and Coronavirus disease-2019 (COVID-19).

11. The nucleic acid molecule according to claim 1, wherein the cancer is one or more selected from the group consisting of solid tumor and blood cancer.

12. The nucleic acid molecule according to claim 11, wherein the cancer is one or more selected from the group consisting of stomach cancer, lung cancer, liver cancer, colorectal cancer, colon cancer, kidney cancer, small intestine cancer, pancreatic cancer, brain tumor, bone cancer, skin cancer, epidermoid carcinoma, squamous cell carcinoma, breast cancer, sclerosing gonadosis, head and neck cancer, esophagus cancer, pharynx cancer, thyroid cancer, parathyroid cancer, neuroblastoma, melanoma, sarcoma, prostate cancer, uterine cancer, cervical cancer, ovarian cancer, vaginal cancer, vulvar cancer, urethral cancer, bladder cancer, penile cancer, testicular cancer, hematological cancer, angiosarcoma, leukemia, lymphoma, fibroadenoma, and metastatic cancer thereof.

13. A vaccine composition for preventing or treating viral infection or cancer, comprising the nucleic acid molecule according to claim 1.

14. The vaccine composition according to claim 13, wherein the nucleic acid molecule is encapsulated in or linked to a delivery vehicle.

15. The vaccine composition according to claim 14, wherein the delivery vehicle is one or more selected from the group consisting of liposome-based delivery vehicle, lipid-based delivery vehicle, polymer-based delivery vehicle and lipid-polymer hybrid nanoparticle.

16. The vaccine composition according to claim 15, wherein the delivery vehicle is one or more selected from the group consisting of liposome, phytosome, ethosome, lipid nanoparticle, lipid-like nanoparticle, lipid emulsion, lipoplex and lipid micelle, polymersome, polymeric nanoparticle, dendrimer, nanosphere, polyplex, polymeric micelle, lipid-polymer hybrid nanoparticle, cationic nanoemulsion, anionic nanoemulsion and lipopolyplex.
